# EUROPEAN PATENT APPLICATION

(11) **EP 4 644 542 A1**
(43) Date of publication of application: **05.11.2025**
(21) Application number: 23912944.8
(22) Date of filing: 27.12.2023
(51) Int. Cl.: C12N 9/90, C12N 15/70, C12P 19/02

(54) **MUTANT ALLULOSE-3-EPIMERASE AND USE THEREOF**

(30) Priority: 30.12.2022 KR 20220190879
(71) Applicant: Samyang Corporation, Jongno-gu Seoul 03129 (KR)
(72) Inventor: KIM, Jeongmin, Yongin-si, Gyeonggi-do 16826 (KR); LEE, Sanghee, Gwangju-si, Gyeonggi-do 12788 (KR); PARK, Busoo, Hanam-si, Gyeonggi-do 13014 (KR); KWON, Soungyu, Gwangmyeong-si, Gyeonggi-do 14311 (KR); AHN, Sinhye, Goyang-si, Gyeonggi-do 10374 (KR)
(74) Representative: Vossius & Partner Patentanwälte Rechtsanwälte mbB
(86) International application number: PCT/KR2023/021763
(87) International publication number: WO 2024/144270

(57) **Abstract**

The present invention relates to: an enzyme protein comprising a mutated amino acid sequence and having the fructose-to-allulose conversion activity; a recombinant microorganism including a gene encoding the enzyme protein; a composition for producing allulose comprising the recombinant microorganism; and a method of producing allulose by using the recombinant microorganism.

## Description

### Technical Field

The present invention provides a mutant allulose-3-epimerase protein, a recombinant microorganism and/or a composition for producing allulose including the same, and/or a method of producing allulose using the same.

### Background Art

Glucose is used as a source of energy when ingested into the body and therefore is an essential sugar. However, excessive intake of glucose causes adult diseases such as obesity and diabetes. In this regard, a recently emerging sugar material to overcome this issue is allulose (e.g., D-allulose, D-psicose).

Allulose is produced by epimerization of D-fructose and is low in calories by having a structure that makes it difficult to be used as an energy source when ingested. In this regard, allulose has the function of inhibiting the absorption of glucose, so it can be used as a sugar effective against diabetes by preventing blood sugar levels from rising. In addition, it has the function of a sugar that is also effective against obesity because it prevents fat accumulation by inhibiting enzyme reactions involved in fat accumulation. Besides, allulose can be converted into allose with anticancer effects, and has been reported to exhibit similar effects on its own, so it is a sugar material that is expected to see expanded use as a sugar with health functionality.

In addition, allulose has 70 % sweetness compared to sugar, and in this regard, it is a sugar that can maintain an appropriate level of sweetness when eaten. Therefore, it has recently been used as a functional sugar and is often used in low-calorie beverages.

Since the functionality of allulose was confirmed, research has continued on methods of producing allulose by conversion of fructose. In addition, research is also being conducted on methods of producing allulose by using glucose, which has a lower cost than fructose. Regarding the conversion of fructose to allulose, studies on use of allulose epimerase derived from *Agrobacterium tumefaciense, Clostridium cellulolyticum, Clostridium boleae,* and the like, tagatose epimerase derived from *Pseudomonas chichori, Rhodobacter sphaeroides,* and the like, and enzymes derived from *Arthrobacter gloformis, Staphylococcus aureus, Mesorhizobium loti, Methylonmonus sp.* and the like for the conversion are being studied. Recently, the production of allulose by multienzyme conversion using glucose or starch has also been studies in terms of reducing the cost of allulose production.

Allulose is generally produced by conversion through epimerization of the hydroxyl moiety located at the third carbon of fructose. In an enzymatic reaction, the conversion ratio between fructose and the substrate usually reaches equilibrium in a ratio of 70:30. Therefore, the conversion rate of allulose generally reaches a maximum of approximately 30%.

However, many reports have confirmed that increasing the temperature of the conversion reaction increases the conversion rate of allulose. Therefore, enhancing the thermal stability of an enzyme can improve its overall stability, making it possible to increase the reaction temperature and thereby improve the conversion yield.

Accordingly, the present study aimed to identify mutant enzymes with increased thermal stability by studying mutations that increase the thermal stability of the enzymes and to increase allulose productivity through increased activity and thermal stability.

### Description

### Technical Problem

An aspect of the present invention relates to a mutant allulose-3-epimerase protein having enhanced allulose conversion activity and thermal stability.

Another aspect of the present invention relates to a nucleic acid sequence encoding the mutant allulose-3-epimerase protein.

Another aspect of the present invention relates to a recombinant microorganism including the nucleic acid sequence encoding the mutant allulose-3-epimerase protein.

Another aspect of the present invention relates to a composition for producing allulose using the mutant allulose-3-epimerase protein and/or the recombinant microorganism.

Another aspect of the present invention relates to a method of producing allulose from a substrate using the mutant allulose-3-epimerase protein and/or the recombinant microorganism.

### Technical Solution

An aspect of the present invention provides a mutant allulose-3-epimerase protein in which one or more amino acids in the amino acid sequence of SEQ ID NO: 1 are substituted.

Specifically, the mutant allulose-3-epimerase protein may be a mutant allulose-3-epimerase protein in which at least one amino acid selected from the group consisting of amino acids at positions 23, 75, 115, 126, 255, 267, and 269 from the N-terminus of the amino acid sequence of SEQ ID NO: 1 is

substituted with at least one amino acid selected from the group consisting of isoleucine (I), glutamic acid (E), glycine (G), valine (V), alanine (A), and lysine (K).

Another aspect of the present invention provides a polynucleotide encoding the allulose-3-epimerase protein of the present invention, and
specifically provides a polynucleotide including a nucleic acid sequence encoding an amino acid sequence in which at least one amino acid selected from the group consisting of amino acids at positions 23, 75, 115, 126, 255, 267, and 269 from the N-terminus of the amino acid sequence of SEQ ID NO: 1 is
with at least one amino acid selected from the group consisting of isoleucine (I), glutamic acid (E), glycine (G), valine (V), alanine (A), and lysine (K).

Another aspect of the present invention provides a recombinant vector including the polynucleotide of the present invention.

Another aspect of the present invention provides a recombinant microorganism including a gene encoding the allulose-3-epimerase protein of the present invention. The microorganism may be at least one microorganism selected from the group consisting of a strain of the genus Escherichia (e.g., *Escherichia coli),* a strain of the genus Bacillus (e.g., *Bacillus subtilis*), a strain of the genus Corynebacterium (e.g., *Corynebacterium glutamicum*), a strain of the genus Saccharomyces (e.g., *Saccharomyces cerevisiae*), and a strain of the genus Pichia (e.g., *Pichia pastoris*), but is not limited thereto.

Another aspect of the present invention provides a composition for producing allulose, including at least one selected from the group consisting of the allulose-3-epimerase protein of the present invention and microbial cells of a recombinant microorganism expressing the allulose-3-epimerase protein, a lysate of the microbial cells of the microorganism, a culture solution of the microorganism, and an extract of the foregoing.

Another aspect of the present invention provides a method of producing allulose, including reacting a substrate with at least one selected from the group consisting of the allulose-3-epimerase protein, a recombinant microorganism expressing the allulose-3-epimerase protein, microbial cells of the microorganism, a lysate of the microbial cells of the microorganism, a culture of the microorganism, and an extract of the foregoing.

The enzyme protein of the present invention may include a sequence in which at least one amino acid selected from the group consisting of amino acids at positions 23, 75, 115, 126, 255, 267, and 269 from the N-terminus of the amino acid sequence of SEQ ID NO: 1 is

substituted with at least one amino acid selected from the group consisting of isoleucine (I), glutamic acid (E), glycine (G), valine (V), alanine (A), and lysine (K), and may have enhanced allulose conversion activity and thermal stability compared to a wild-type enzyme.

The recombinant microorganism including a gene encoding the enzyme protein according to the present invention may include an amino acid sequence in which at least one amino acid selected from the group consisting of amino acids at positions 23, 75, 115, 126, 255, 267, and 269 from the N-terminus in the amino acid sequence of SEQ ID NO: 1 is

substituted with at least one amino acid selected from the group consisting of isoleucine (I), glutamic acid (E), glycine (G), valine (V), alanine (A), and lysine (K), and may have higher allulose conversion activity and thermal stability compared to a microorganism including a gene encoding an enzyme protein not having such amino acid mutations (e.g., a wild-type strain).

Hereinafter, the present invention will be described in more detail.

In an embodiment of the present invention, there is provided an allulose-3-epimerase protein in which at least one amino acid selected from the group consisting of amino acids at positions 23, 75, 115, 126, 255, 267, and 269 from the N-terminus in the amino acid sequence of SEQ ID NO: 1 is substituted with at least one amino acid selected from the group consisting of isoleucine (I), glutamic acid (E), glycine (G), valine (V), and alanine (A).

Specifically, the substituted amino acid sequence may include, although not limited thereto, at least one substitution selected from the group consisting of a substitution of alanine at position 23 with isoleucine, a substitution of serine at position 75 with glutamic acid, a substitution of aspartic acid at position 115 with glycine, a substitution of asparagine at position 126 with valine, a substitution of leucine at position 255 with valine, a substitution of glutamic acid at position 267 with lysine, and a substitution of glycine at position 269 with alanine,
wherein the positions are numbered from the N-terminus of the amino acid sequence of SEQ ID NO: 1.

In addition, the substituted amino acid sequence may include: although not limited thereto, at least one amino acid substitution selected from the group consisting of a substitution of alanine at position 23 with isoleucine, a substitution of serine at position 75 with glutamic acid, a substitution of aspartic acid at position 115 with glycine, a substitution of asparagine at position 126 with valine, a substitution of leucine at position 255 with valine, and a substitution of glycine at position 269 with alanine; and
a substitution of glutamic acid at position 267 with lysine,
wherein the positions are numbered from the N-terminus of the amino acid sequence of SEQ ID NO: 1.

An embodiment of the present invention provides an allulose-3-epimerase protein having an amino acid sequence with at least 80 % sequence identity, at least 85 % sequence identity, at least 90 % sequence identity, at least 95 % sequence identity, at least 96 % sequence identity, at least 97 % sequence identity, at least 98 % sequence identity, at least 99 % sequence identity, or at least 99.3 % sequence identity to the amino acid sequence of SEQ ID NO: 1.

Specifically, the allulose-3-epimerase protein of the present invention, in which at least one amino acid selected from the group consisting of amino acids at positions 23, 75, 115, 126, 255, 267, and 269 from the N-terminus in the amino acid sequence of SEQ ID NO: 1 is substituted with at least one amino acid selected from the group consisting of isoleucine (I), glutamic acid (E), glycine (G), valine (V), and alanine (A), may have 80 % or more sequence identity, 85 % or more sequence identity, 90 % or more sequence identity, 95 % or more sequence identity, 96 % or more sequence identity, 97 % or more sequence identity, 98 % or more sequence identity, 99 % or more sequence identity, or 99.3 % or more sequence identity to the amino acid sequence of SEQ ID NO: 1, provided that an enzyme protein having the amino acid sequence of SEQ ID NO: 1, i.e., a wild-type enzyme protein not having a mutation may be excluded.

An embodiment of the present invention provides an allulose-3-epimerase protein having (i) a sequence in which at least one amino acid selected from the group consisting of amino acids at positions 23, 75, 115, 126, 255, 267, and 269 from the N-terminus in the amino acid sequence of SEQ ID NO: 1 is substituted with at least one amino acid selected from the group consisting of isoleucine (I), glutamic acid (E), glycine (G), valine (V), and alanine (A) or with the amino acid sequence of SEQ ID NO: 1; or (ii) an amino acid sequence with 80 % or more sequence identity, 85 % or more sequence identity, 90 % or more sequence identity, 95 % or more sequence identity, 96 % or more sequence identity, 97 % or more sequence identity, 98 % or more sequence identity, 99 % or more sequence identity, or 99.3 % or more sequence identity to the amino acid sequence of SEQ ID NO: 1, provided that an enzyme protein having the amino acid sequence of SEQ ID NO: 1, i.e., a wild-type enzyme protein not having a mutation may be excluded.

The allulose conversion activity in enzymatic reactions of the enzyme protein of the present invention may be measured by using microbial cells expressing the enzyme, a culture solution in which the microbial cells are cultured, and/or a supernatant obtained by centrifugation of the culture solution. Specifically, it may be measured by using microbial cells obtained by a reaction between microbial cells expressing the enzyme and a substrate, a culture solution in which the obtained microbial cells are cultured, and/or a supernatant obtained by centrifugation of the culture solution. The substrate may include one or more selected from the group consisting of fructose, allulose, tagatose, xylose, sorbose, ribulose, and ketose, but is not limited thereto.

The microbial cell used to confirm the allulose conversion activity in enzymatic reactions of the enzyme protein may be a cell of a strain of the genus Escherichia (e.g., *Escherichia coli),* but is not limited thereto.

In a substrate conversion reaction using the mutant enzyme protein or a microbial cell producing the same, an allulose conversion activity of the enzyme protein of the present invention or the enzyme protein of the composition for producing allulose of the present invention may be 105 % or more, 110 % or more, 115 % or more, 120 % or more, 125 % or more, 130 % or more, 135 % or more, 140 % or more, 145 % or more, 150 % or more, 155 % or more, or 160 % or more, 105 % to 200 %, 105 % to 180 %, 105 % to 170 %, 105 % to 165 %, 110 % to 200 %, 110 % to 180 %, 110 % to 170 %, 110 % to 165 %, 120 % to 200 %, 120 % to 180 %, 120 % to 170 %, 120 % to 165 %, 130 % to 200 %, 130 % to 180 %, 130 % to 170 %, 130 % to 165 %, 140 % to 200 %, 140 % to 180 %, 140 % to 170 %, 140 % to 165 %, 150 % to 200 %, 150 % to 180 %, 150 % to 170 %, 150 % to 165 %, 160 % to 200 %, 160 % to 180 %, 160 % to 170 %, or 160 % to 165 %, or for example, 107.16 %, 116.69 %, 124.69 %, 136.67 %, 140 %, 151.97 %, 159.88 %, 162.01 %, 162.04 %, or 167.27 %, relative to the allulose conversion activity of a wild-type enzyme protein comprising the amino acid sequence of SEQ ID NO: 1, which is defined as 100%. The evaluation of enzyme activity using the enzyme protein or the microbial cell producing the enzyme may be conducted by performing a substrate conversion reaction at 70 °C for 30 minutes using the microbial cell expressing the enzyme protein or by performing a substrate conversion reaction at 70°C for 30 minutes using a purified enzyme obtained by disrupting the microbial cell producing the enzyme.

The thermal stability of the enzyme protein of the present invention or the enzyme protein of the composition for producing allulose of the present invention may be confirmed, although not limited thereto, by comparing the allulose conversion activity after a heat treatment reaction on the microbial cells expressing the enzyme protein, a culture solution in which the microbial cells are cultured, and/or a supernatant obtained by centrifugation of the culture solution, with the allulose conversion activity before the heat treatment, or by measuring the half-life of the enzyme protein, defined as the time required for the allulose conversion activity to decrease to 50%, relative to the allulose conversion activity before the heat treatment which is defined as 100%.

The microbial cell used to confirm the allulose conversion activity in enzymatic reactions of the enzyme protein may be a cell of a strain of the genus Escherichia (e.g., *Escherichia coli),* but is not limited thereto.

The confirmation of thermal stability may be achieved by performing heat treatment at 60 °C or 70 °C for 1 hour, 2 hours, 3 hours, 4 hours, 5 hours, 6 hours, 7 hours, 7.5 hours, 8 hours, 9 hours, 10 hours, or 11 hours using the microbial cell expressing the enzyme protein or using a purified enzyme obtained by disrupting the microbial cell producing the enzyme.

The half-life (hr) of the enzyme protein of the present invention or the enzyme protein of the composition for producing allulose of the present invention under heat treatment condition at 60 °C may be, although not limited thereto, 130 % or more, 150 % or more, 200 % or more, 250 % or more, 300 % or more, 350 % or more, 400 % or more, 130 % to 500 %, 130 % to 450 %, 130 % to 420 %, 200 % to 500 %, 200 % to 450 %, 200 % to 420 %, 300 % to 500 %, 300 % to 450 %, 300 % to 420 %, 350 % to 500 %, 350 % to 450 %, 350 % to 420 %, 380 % to 500 %, 380 % to 450 %, or 380 % to 420 %, of for example, 133 % or 400 %, relative to the half-life of the enzyme protein expressed by a microorganism expressing the wild-type enzyme protein including the amino acid sequence of SEQ ID NO: 1, which is defined as 100%.

The half-life (hr) of the enzyme protein of the present invention or the enzyme protein of the composition for producing allulose of the present invention under heat treatment condition at 60 °C may be, although not limited thereto, 2 hours or more, 3 hours or more, 4 hours or more, 5 hours or more, 6 hours or more, 1.7 to 50 hours, 1.7 to 30 hours, 1.7 to 10 hours, 1.7 to 8 hours, 1.7 to 7 hours, 2 to 50 hours, 2 to 30 hours, 2 to 10 hours, 2 to 8 hours, 2 to 7 hours, 3 to 50 hours, 3 to 30 hours, 3 to 10 hours, 3 to 8 hours, 3 to 7 hours, 4 to 50 hours, 4 to 30 hours, 4 to 10 hours, 4 to 8 hours, 4 to 7 hours, 5 to 50 hours, 5 to 30 hours, 5 to 10 hours, 5 to 8 hours, 5 to 7 hours, 6 to 50 hours, 6 to 30 hours, 6 to 10 hours, 6 to 8 hours, or 6 to 7 hours, or for example, 2 hours or 6 hours.

The enzyme protein of which the half-life was confirmed under heat treatment condition at 60 °C may be an allulose-3-epimerase protein in which,
from the N-terminus of the amino acid sequence of SEQ ID NO: 1,
at least one amino acid selected from the group consisting of amino acids at positions 23, 75, 115, 126, 255, 267, and 269 is
substituted with at least one amino acid selected from the group consisting of isoleucine (I), glutamic acid (E), glycine (G), valine (V), alanine (A), and lysine (K).

The half-life (hr) of the enzyme protein of the present invention or the enzyme protein of the composition for producing allulose of the present invention under heat treatment condition at 70 °C may be, although not limited thereto, 0.5 hour or more, 2 hours or more, 4 hours or more, 6 hours or more, 8 hours or more, 10 hours or more, 0.5 to 20 hours, 0.5 to 15 hours, 0.5 to 12 hours, 1 to 20 hours, 1 to 15 hours, 1 to 12 hours, 5 to 20 hours, 5 to 15 hours, 5 to 12 hours, 8 to 20 hours, 8 to 15 hours, or 8 to 12 hours, or for example, 0.67 hours or 10 hours.

The allulose conversion activity after the heat treatment reaction at 70 °C for 7 hours of the enzyme protein of the present invention or the enzyme protein of the composition for producing allulose of the present invention may be 60 % to 100 %, or 65 % to 100 %, or for example, 65.57 %, relative to the allulose conversion activity of the enzyme protein before the heat treatment reaction which is defined as 100%, but is not limited thereto.

The enzyme protein of which the half-life was confirmed under heat treatment conditions of 70 °C may be an allulose-3-epimerase protein in which,
from the N-terminus of the amino acid sequence of SEQ ID NO: 1,
at least one amino acid selected from the group consisting of amino acids at positions 23, 75, 115, 126, 255, 267, and 269 is
substituted with at least one amino acid selected from the group consisting of isoleucine (I), glutamic acid (E), glycine (G), valine (V), alanine (A), and lysine (K), and specifically, may be
an allulose-3-epimerase protein including: at least one amino acid substitution selected from the group consisting of a substitution of alanine at position 23 with isoleucine, a substitution of serine at position 75 with glutamic acid, a substitution of aspartic acid at position 115 with glycine, a substitution of asparagine at position 126 with valine, a substitution of leucine at position 255 with valine, and a substitution of glycine at position 269 with alanine; and
a substitution of glutamic acid at position 267 with lysine,
wherein the positions are numbered from the N-terminus of the amino acid sequence of SEQ ID NO: 1.

The enzyme protein of the present invention may be activated by metal ions, and thus, when producing allulose by using a strain of the genus *Microbacterium,* the conversion efficiency from fructose to allulose, i.e., the allulose yield, may be increased by adding metal ions. Accordingly, a composition for producing allulose including one or more selected from the group consisting of the enzyme protein, a recombinant microorganism expressing the enzyme protein, and microbial cells of the microorganism, a lysate of the microbial cells of the microorganism, a culture solution of the microorganism, and an extract of the foregoing, or a composition for producing allulose by using a microorganism producing the enzyme protein may further include metal ions. In addition, the method of producing allulose by using the enzyme protein may further include adding a metal ion.

The metal ion may be one or more selected from the group consisting of a manganese ion, a magnesium ion, a nickel ion, a cobalt ion, and the like, and in an embodiment, the metal ion may be a manganese ion, a cobalt ion, or a mixture thereof. In the presence of a manganese ion, a cobalt ion, or a mixture thereof as the metal ion, the allulose conversion activity may be increased by 1.2-fold, 1.3-fold, 1.4-fold, 1.5-fold, 1.6-fold, 1.7-fold, 1.8-fold, 1.9-fold, 2.0-fold, 2.1-fold, 2.2-fold, or 2.3-fold or more compared to the absence of the metal ion, or for example, may be increased by 1.2 to 2.3-fold.

The amount of the metal ion added may be in a range of 0.5 mM to 5 mM, 0.5 mM to 4 mM, or 0.5 mM to 3 mM, or for example, 0.5 mM to 2 mM, in consideration of the effect of increasing the yield of allulose production.

Another aspect of the present invention provides a recombinant microorganism including a gene encoding the enzyme protein of the present invention.

The microorganism may be at least one microorganism selected from the group consisting of a strain of the genus Escherichia (e.g., *Escherichia coli),* a strain of the genus Bacillus (e.g., *Bacillus subtilis*), a strain of the genus Corynebacterium (e.g., *Corynebacterium glutamicum*), a strain of the genus Saccharomyces (e.g., *Saccharomyces cerevisiae*), and a strain of the genus Pichia (e.g., *Pichia pastoris*), but is not limited thereto.

Another aspect of the present invention provides a composition for producing allulose, including at least one selected from the group consisting of the enzyme protein of the present invention, the recombinant microorganism expressing the enzyme protein, and microbial cells of the recombinant microorganism expressing the enzyme protein, a lysate of the microbial cells of the microorganism, a culture solution of the microorganism, and an extract of the foregoing.

The culture solution may include an enzyme produced by the recombinant microorganism, and in this regard, may include the microorganism or may be in a cell-free form not including the microorganism. In addition, the lysate may include a lysate obtained by disrupting the microbial cells of the recombinant microorganism or a supernatant obtained by centrifugation of the lysate, and an enzyme produced by the recombinant microorganism.

In the present specification, unless otherwise stated, the recombinant microorganism used for the production of allulose may refer to one or more selected from the group consisting of microbial cells of the microorganism, a culture solution of the strain, and a lysate of the strain.

Another aspect of the present invention provides a method of producing allulose, including causing a substrate to react with at least one selected from the group consisting of the enzyme protein of the present invention, a recombinant microorganism expressing the enzyme protein, and microbial cells of the microorganism, a lysate of the microbial cells of the microorganism, a culture solution of the microorganism, and an extract of the foregoing.

The substrate may include one or more selected from the group consisting of fructose, allulose, tagatose, xylose, sorbose, ribulose, and ketose, but is not limited thereto.

The method of producing allulose may further include separating and/or purifying allulose from the reaction product. The culture solution may be a culture solution resulting from a reaction between a substrate with one or more selected from the group consisting of the recombinant microorganism, microbial cells of the recombinant microorganism, microbial cells of the recombinant microorganism, a lysate of the microbial cells of the microorganism, a culture of the microorganism, and an extract of the foregoing, but is not limited thereto.

Specifically, the method of producing allulose of the present invention may comprise separating an allulose reaction product, including separating a product of the allulose conversion reaction by ion purification and simulated moving bed (SMB) chromatography processes. In a specific embodiment, the product of the allulose conversion reaction is subjected to the SMB chromatography to separate into an allulose fraction having a higher allulose content than the product of the conversion reaction and a fructose raffinate, and the allulose fraction may be produced as a liquid syrup by an allulose concentration process, or as an allulose crystal by an allulose crystallization process. The allulose crystallization process may include: performing secondary ion purification of the allulose fraction obtained in the high-purity separation process; concentrating the ion-purified allulose fraction; and obtaining allulose crystals and allulose crystallization mother liquor by crystallizing allulose from the concentrates.

### Advantageous Effects

The present invention relates to an enzyme protein having allulose-3-epimerization activity in which a specific amino acid is mutated, a recombinant microorganism including the enzyme protein, a composition for producing allulose including the enzyme protein, or a method of producing allulose using the enzyme protein, wherein the enzyme protein has high conversion activity from fructose to allulose and excellent thermal stability.

### Description of Drawings

FIG. 1 is a schematic diagram of a recombinant vector into which a D-allulose-3-epimerase gene according to an example of the present invention is cloned.
FIG. 2 is a schematic diagram of a recombinant vector into which a D-allulose-3-epimerase gene for enzyme purification is cloned.

### Mode for Invention

The present invention will be described in more detail with reference to the following examples, but the scope of the invention is not intended to be limited by the following examples.

### Example 1. Cloning of D-allulose-3-epimerase gene

### Example 1-1. Preparation of enzyme gene

A D-allulose-3-epimerase (D-tagatose 3-epimerase) identified from *Microbacterium foliorum* (SY27B-MF; Accession No: KCCM11774P) was expressed in *Escherichia coli* and its enzyme activity was confirmed, and its DNA was synthesized to find a variant strain with improved thermostability. To ensure proper expression in *E. coli,* the amino acid sequence of the protein was synthesized with codons optimized for *E. coli.*

The gene sequence obtained through codon optimization was synthesized by Integrated DNA Technologies (IDT, USA) upon request. The synthesized gene was then named MDPE, and the information on the corresponding gene with the codon optimization is shown in Table 1 below. The amino acid sequence of the MDPE gene corresponds to the sequence of SEQ ID NO: 1, and the base sequence of the MDPE gene corresponds to the sequence of SEQ ID NO: 2.

**[Table 1]**

| Type | Sequence (5'→3') | SEQ ID NO: |
|---|---|---|
| MDPE_W ild_AA | MNIGCHGLVWTGHFDADGIRLSAEQTKAAGFDLIEFPLM DPFTFDVAAAKEALEEHDLAVSASLGLSGATDVTSSDPA VVAAGEALLMKAVDVLAELGGRHFCGVIYSAMQKYMDP VTTEGLESSRRTIARVADHAAERGISVSLEVVNRYETNVL NTARQALAYLAEVDRPNLGIHLDTYHMNIEESDMFAPVL DAAPALRYVHIGESHRGYLGTGTVDFDNFFKALGRIGYD GPIVFESFSSAVVAPDLSRMLGIWRNLWTDNVELGAHAN AYIRDKLVAVDSIRLH | 1 |
| MDPE_W ild_DNA | ATGAACATCGGTTGCCACGGTCTGGTTTGGACCGGTC ACTTCGACGCGGACGGTATCCGTCTGTCTGCGGAACA GACCAAAGCGGCGGGTTTCGACCTGATCGAATTCCCG CTGATGGACCCGTTCACCTTCGACGTTGCGGCGGCGA AAGAAGCGCTGGAAGAACACGACCTGGCGGTTTCTGC GTCTCTGGGTCTGTCTGGTGCGACCGACGTTACCTCT TCTGACCCGGCGGTTGTTGCGGCGGGTGAAGCGCTG CTGATGAAAGCGGTTGACGTTCTGGCGGAACTGGGTG GTCGTCACTTCTGCGGTGTTATCTACTCTGCGATGCA GAAATACATGGACCCGGTTACCACCGAAGGTCTGGAA TCTTCTCGTCGTACCATCGCGCGTGTTGCGGACCACG CGGCGGAACGTGGTATCTCTGTTTCTCTGGAAGTTGT TAACCGTTACGAAACCAACGTTCTGAACACCGCGCGT | 2 |
| | CAGGCGCTGGCGTACCTGGCGGAAGTTGACCGTCCG AACCTGGGTATCCACCTGGACACCTACCACATGAACA TCGAAGAATCTGACATGTTCGCGCCGGTTCTGGACGC GGCGCCGGCGCTGCGTTACGTTCACATCGGTGAATCT CACCGTGGTTACCTGGGTACCGGTACCGTTGACTTCG ACAACTTCTTCAAAGCGCTGGGTCGTATCGGTTACGA CGGTCCGATCGTTTTCGAATCTTTCTCTTCTGCGGTTG TTGCGCCGGACCTGTCTCGTATGCTGGGTATCTGGCG TAACCTGTGGACCGACAACGTTGAACTGGGTGCGCAC GCGAACGCGTACATCCGTGACAAACTGGTTGCGGTTG ACTCTATCCGTCTGCAC | |

### Example 1-2. Production of a recombinant microorganism including enzyme gene

A polynucleotide of the MDPE of Example 1-1, which was synthesized by codon optimization for proper expression in E. *coli,* was amplified by polymerase chain reaction (PCR). Specifically, a reaction solution for gene amplification (Polymerase Chain Reaction, PCR) with a final volume of 50 µl containing 50 ng of the synthesized MDPE gene, 10 pmol of forward primer (base sequence of SEQ ID NO: 3) and 10 pmol of reverse primer (base sequence of SEQ ID NO: 4) for each amino acid residue, 1 µl of Phusion polymerase, 10 µl of reaction buffer (5X), and 1 µl of 10 mM dNTP was prepared and amplified by PCR, and the sequence information of the primers used herein is shown in Table 2 below.

**[Table 2]**

| Type | Base sequence (5'→3') | SEQ ID NO: |
|---|---|---|
| MDPE_Cloning _For_Primer | ccatgattacgccaagcttATGAACATCGGTTGCC ACG | 3 |
| MDPE_Cloning _Rev_Primer | CGGTACCCGGGGATCCTAGCAGCCGGAT CTCAGTGCAGACGGATAGAGTC | 4 |

Specifically, the PCR was performed with the GeneAmp PCR system 9700 using the PCR solution, including one cycle at 98 °C (30 seconds), 18 cycles of [98 °C (10 seconds), 55 °C (30 seconds), and 72 °C (2 minutes and 30 seconds)], and one cycle at 72 °C (5 minutes), thereby obtaining a large amount of genes after the gene amplification.

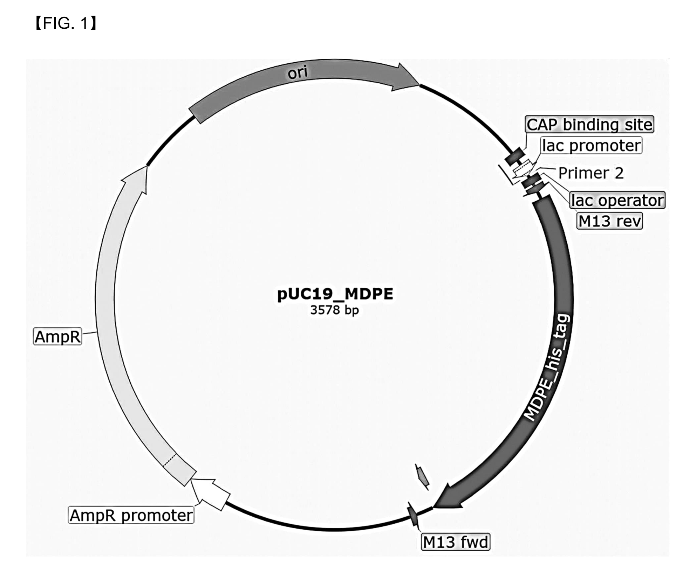

A pUC19 vector (NEB, New England Biolab) was cut using restriction enzymes, Hindlll and BamHI, and the amplified gene and the pUC19 vector were ligated at the restriction enzyme sites by using 2XHiFi DNA master mix (NEB) to produce a pUC19/allulose-3-epimerase recombinant vector (pUC19_MDPE), and a schematic diagram of the produced recombinant vector is shown in FIG. 1. The produced recombinant vector was transformed into E. *coli* DH10b competent cells (TransGen, Trans10) according to a heat shock method, thereby producing a recombinant microorganism (DH10b pUC19_MDPE).

### Example 2. Mutagenesis of D-allulose-3-epimerase gene

The amino acids, which were selected based on analysis of the amino acid sequences between homologous genes and 3D structure model analysis of active sites and metal-binding sites, were substituted with other amino acids, and these recombinant mutant enzymes were produced in E. *coli* and analyzed for changes in the allulose epimerase activity.

### Example 2-1. Site-directed mutagenesis (SDM)

The sequenced D-allulose-3-epimerases derived from *Arthrobacter globiformis, Agrobacterium tumefaciense, Clostridium cellulolyticum,* and *Methylomonus sp.* among epimerases and their sequences were analyzed to identify homology sites, and mutation sites were then identified according to the homology sites.

Specifically, as a result of comparing the homology of the epimerases, the amino acid at the 23rd position in the D-allulose-3-epimerases derived from *Agrobacterium tumefaciense, Clostridium cellulolyticum,* and *Methylomonus sp* was confirmed to be isoleucine (I), and thus this position was selected as a mutation site. In addition, by comparing with the **D-allulose-3-epimerase** derived from *Arthrobacter globiformis,* the amino acid at the 75th position was confirmed to be glutamic acid (E), and thus this position was also selected as a mutation site. These sites were confirmed by using the database of the Protein Data Bank to ensure that the enzyme is structurally active.

Such substitution mutations in the MDPE were produced by site-directed mutagenesis (SDM) using Quikchange^{®} (Stratagene) applied from the prior studies. The Quikchange^{®} SDM of Stratagene utilizes PCR to amplify the entire plasmid DNA and uses Dpnl enzyme to digest the template DNA, rather than amplifying genes corresponding to enzyme sites by PCR and subcloning the amplified genes into plasmid DNA. The Quikchange^{®} SDM has the advantage of being able to induce mutations more quickly.

The pUC19_MDPE of Example 1-2 was used as the template DNA for replication for substitution mutation of the MDPE of Example 1. A reaction solution for gene amplification (e.g., PCR) with a final volume of 50 µl containing 10 pmol of forward primer (base sequence of SEQ ID NO: 5 or 7) and 10 pmol of reverse primer (base sequence of SEQ ID NO: 6 or 8) for each amino acid residue, 1 µl of Phusion polymerase, 10 µl of reaction buffer (5X), and 1 µl of 10 mM dNTP was prepared and amplified by PCR, and the information of the primers used herein is shown in Table 3 below.

**[Table 3]**

| Type | Base sequence (5'→3') | SEQ ID NO: |
|---|---|---|
| MDPE_A23I_Clonin g_For_Primer | GACGGTATCCGTCTGTCTATTGAA CAGACCAAAGCGGC | 5 |
| MDPE_A23I_Clonin g_Rev_Primer | GCCGCTTTGGTCTGTTCAATAGAC AGACGGATACCGTC | 6 |
| MDPE_S75E_Cloni ng_For_Primer | CGACCGACGTTACCTCTGAAGAC CCGGCGGTTG | 7 |
| MDPE_S75E_Cloni ng_Rev_Primer | CAACCGCCGGGTCTTCAGAGGTA ACGTCGGTCG | 8 |
| MDPE_Mut_For_Pri mer | gaccatgattacgccaagcttatg | 9 |
| MDPE_Mut_Rev_Primer | cggtacccggggatcctagcagccggatctcaGTG | 10 |

Specifically, the PCR was performed with the GeneAmp PCR system 9700 using the PCR solution, including one cycle at 98 °C (30 seconds), 18 cycles of [98 °C (10 seconds), 55 °C (30 seconds), and 72 °C (2 minutes and 30 seconds)], and one cycle at 72 °C (5 minutes), thereby obtaining a large amount of genes after the gene amplification.

1 µl of Dpnl (10 Unit/µl, NEB), which is an enzyme that recognizes and digests the non-mutated template DNA, was added to 50 µl volume of the reaction solution at the end of the PCR and treated at 37 °C for 3 hours. Following the treatment, in substantially the same manner as in the method of producing the recombinant strain DH10b pUC19_MDPE of Example 1-2, the pUC19_MDPE_A23I gene (A23I mutation) that was PCR-treated with MDPE_A23I_Cloning primer and the pUC19_MDPE_S75E gene (S75E mutation) that was PCR-treated by with MDPE_S75E_Cloning primer were each transformed into the E. *coli* DH10b, thereby obtaining a DH10b pUC19_MDPE_A23I recombinant strain and a DH10b pUC19_MDPE_S75E recombinant strain.

To identify enzyme mutations in the obtained recombinant strains, comparison was made by using the NCBI website (BLAST) tool and amino acid sequence alignment of the wild-type enzyme.

### Example 2-2. Mutations via error prone PCR

Mutants were produced via error prone PCR, which induces mutations during a gene amplification process. Specifically, to produce mutants, a reaction solution for gene amplification (e.g., PCR) with a final volume of 50 µl containing 10 ng of template DNA (pUC19_MDPE), 10 pmol of forward primer (base sequence of SEQ ID NO: 9) and 10 pmol of reverse primer (base sequence of SEQ ID NO: 10) for each amino acid residue, 1 µl of Taq polymerase (Clontech), 5 µl of reaction buffer (10X TITANIUM Taq buffer), 1 µl of diversify dNTP mix, 1 µl of dGTP (2 mM), and 4 µl of MnSO₄ was prepared with addition of PCR Grade water, and then amplified by PCR. The information of the primers used herein is shown in Table 3 above.

Specifically, the PCR was performed on the GeneAmp PCR system 9700 using the PCR solution with an initial cycle at 94 °C for 30 seconds, followed by 25 cycles of 94 °C for 30 seconds and 68 °C for 1 minute, and then a final reaction at 68 °C for 1 minute.

The gene amplified by mutagenesis was subjected to electrophoresis to obtain a band at a desired location, and the obtained gene band was extracted. Then, in substantially the same manner as in the method of producing the recombinant strain DH10b pUC19_MDPE of Example 1-2, the PCR-treated gene was transformed into E. *coli* DH10b to obtain a recombinant microorganism.

### Example 2-3. Mutation screening using high-throughput-screening (HTS)

The transformed recombinant microorganism of Example 2-2 was obtained by smearing on an LB ampicillin solid medium supplemented with 10 g/L of Bacto Tryptone (Difco), 5 g/L of yeast extract (Difco), 10 g/L of NaCl (Daejung Chemical), 100 mg/L of ampicillin (Sigma), and 20 g/L of Bactor Agar. The colonies thus obtained were subjected to first screening using HTS.

After the first screening, 300 µL of LB-ampicillin liquid medium (supplemented with 10 g/L of Bacto Tryptone (Difco), 5 g/L of yeast extract (Difco), 10 g/L of NaCl (Daejung Chemical), and 100 mg/L of ampicillin (Sigma)) was inoculated into a 96-well multi-colony culture vessel (Greiner) and cultured with shaking at 37 °C and 200 rpm for 16 to 18 hours.

The enzyme activity of the shake-cultured microbial cells was confirmed by fructose-dehydrogenase assay. The assay method is as follows: 75 µL of the culture solution obtained by the shaking culture was transferred to a 96-well plate, an equal amount of 100 mM allulose substrate (100 mM Mcilvaine buffer pH 6.0) was added thereto, and a microbial cell reaction was carried out at 60°C for 30 minutes.

After transferring 10 µL of the reaction solution in which fructose was generated from allulose through the reaction to a new 96-well plate, reaction solution A (150 mM Mcilvain buffer pH 4.5), reaction solution B (0.1 M potassium ferricyanide, 0.1% Triton X-100), and reaction solution C (0.1 M potassium ferricyanide, 0.1% Triton X-100, 0.05% BSA) were mixed in a volume ratio of 70:10:10, and 90 µL of the mixed solution was added to the reaction solution containing the converted fructose.

Then, after incubation at 37 °C for 30 minutes, 50 µL of reaction solution D (1.25 mM Iron (III) sulfate hydrate, 0.3 % SDS, 8 % phosphoric acid) was added, followed by incubation at 37 °C for 20 minutes to stop the reaction. After stopping the reaction, the absorbance at 660 nm was measured to select strains with high absorbance compared to the recombinant strain DH10b pUC19_MDPE of Example 1-2 which did not undergo any enzyme mutation.

### Example 2-4. Identification of mutant amino acid residues in mutant protein

Among the recombinant microorganism obtained in Example 2-1 and the mutations obtained by the error-prone PCR in Example 202, amino acid residue mutations in the enzyme selected in Example 2-3 were identified.

Specifically, after purifying the plasmid by using a plasmid prep kit (GENALL), the gene sequence of the subcloned epimerase portion of the plasmid was transcribed into the amino acid sequence of the gene sequence by using a sequence analysis service (Macrogen), and the mutation location was confirmed by comparing it with the amino acid sequence of the wild-type enzyme. The information of primers used is shown in Table 4 below.

**[Table 4]**

| Type | Sequence (5'->3') | SEQ ID NO: |
|---|---|---|
| M13F_Primer | GCGGATAACAATTTCACACAGG | 11 |
| M13R_Primer | GTAAAACGACGGCCAGT | 12 |

Among the enzymes in the recombinant strains, strains containing enzymes with A23I, S75E, D115G, R126V, L255V, and G269A mutations were first selected to prepare DH10b pUC19_MDPE_A23I (A23I mutation), DH10b pUC19_MDPE_S75E (S75E mutation), DH10b pUC19_MDPE_D115G (D115G mutation), DH10b pUC19_MDPE_R126V (R126V mutation), DH10b pUC19_MDPE_L255V (L255V mutation), and DH10b pUC19_MDPE_G269A (G269A mutation).

The positions of the mutated amino acids through the reaction were confirmed by generating the three-dimensional structure of the enzyme through a template search of the Protein Data Bank (PDB) by using a protein three-dimensional structure homology model using SWIISS-MODEL (http://www.expasy.org/swissmod/SWIIS-MODEL.html), and the conformation of the three-dimensional structure was confirmed.

### Example 3. Activity evaluation of the first-selected mutant enzymes

### Example 3-1. Enzyme activity confirmation using wild-type enzyme-producing strain

The DH10b pUC19_MDPE strain of Example 1 was cultured by smearing on an LB ampicillin solid medium supplemented with 10 g/L of Bacto Tryptone (Difco), 5 g/L of yeast extract (Difco), 10 g/L of NaCl (Daejung Chemical), 100 mg/L of ampicillin (Sigma), and 20 g/L of Bactor Agar. One colony was taken from the solid medium results and inoculated into 3 ml of LB-ampicillin liquid medium (supplemented with 10 g/L of Bacto Tryptone (Difco), 5 g/L of yeast extract (Difco), 10 g/L of NaCl (Daejung Chemical), and 100 mg/L of ampicillin (Sigma)) and cultured with shaking at 37 °C and 250 rpm for 16 to 18 hours.

After the microbial cells were cultured, the conversion reaction using the microbial cells was performed when the OD₆₀₀ nm reached 2 to 3. Specifically, for the conversion reaction using the microbial cells producing the mutant enzyme, a reaction solution containing 1 mM manganese ion in 50 mM PIPES buffer solution (pH 7.0) so that the concentration of fructose as a reaction substrate was 400 g/L. The concentration of the microbial cells of the strain was set to 1 mg (dcw)/mL, and the reaction was carried out at 70 °C for 30 minutes.

Following the conversion reaction, the supernatant was recovered by centrifugation (13,000 rpm, 15 min) and analyzed by high-performance liquid chromatography (HPLC). The HPLC analysis was performed by using a refractive index detector (RID, Agilent 1280 RID) of HPLC (Agilent, USA) equipped with a Cosmosil Sugar D column. Here, the mobile phase solvent used was 80 % (v/v) acetonitrile, the temperature was 30 °C, and the flow rate was 1.0 mL/min.

The results obtained by HPLC analysis of the supernatant of the microbial culture solution are shown in Table 5 below.

### Example 3-2. Enzyme activity confirmation using mutant enzyme-producing strain

For the strains of Example 2 in which the enzyme mutation was confirmed (DH10b pUC19_MDPE_A23I, DH10b pUC19_MDPE_S75E, DH10b pUC19_MDPE_D115G, DH10b pUC19_MDPE_R126V, DH10b pUC19_MDPE_L255V, or DH10b pUC19_MDPE_G269A), the strains were cultured in substantially the same manner as in Example 3-1, and the products obtained by a substrate conversion reaction were analyzed by HPLC analysis. The results of the analysis are shown in Table 5 below.

In Table 5 below, the substrate conversion activity is expressed as relative conversion activity based on 100% of the substrate conversion activity of the strain producing the wild-type enzyme.

**[Table 5]**

| Strain | Relative conversion activity (%) |
|---|---|
| DH10b pUC19_MDPE (wild-type) | 100.00 |
| DH10b pUC19_MDPE_A23I | 162.04 |
| DH10b pUC19_MDPE_S75E | 159.88 |
| DH10b pUC19_MDPE_D115G | 116.69 |
| DH10b pUC19_MDPE_R126V | 151.97 |
| DH10b pUC19_MDPE_L255V | 124.69 |
| DH10b pUC19_MDPE_G269A | 107.16 |

As a result of confirming the substrate conversion activity of the enzyme using the enzyme-producing strain, it was confirmed that the recombinant strains expressing the A23I, S75E, and R126V mutant enzymes showed increased activity of at least 150 % compared to the recombinant strain expressing the wild-type enzyme, and that the recombinant strain expressing the D115G, L255V, or G269A mutant enzyme showed increased conversion activity of about 107 to 125% compared to the recombinant strain expressing the wild-type enzyme.

### Example 4. Evaluation of thermal stability of strains producing first-selected mutant enzymes

To test the thermal stability of the strain producing the first-selected mutant enzymes, a high-temperature heat treatment was performed on the strains of which the activity was found to be enhanced among the strains in which the enzyme mutations are confirmed in Example 3-2. Specifically, as an experiment to determine the extent to which the enzyme activity was maintained according to the time of treatment at high temperatures, only the strains expressing the mutant enzyme were harvested, suspended in a 50 mM PIPES buffer solution (pH 7.0), and subjected to heat treatment at 60 °C.

Following the heat treatment, a microbial cell reaction was carried out by adding a fructose substrate and manganese ions in substantially the same manner as in Example 3-2, to measure the conversion activity. Based on the conversion activity measured over time, a half-life of the activity was calculated, and the thermal stability of the mutant was evaluated relative to the activity before heat treatment reaction, which was defined as 100%. The half-life was determined as the time point at which the activity of the mutant strain decreased to 50%.

The half-life measurements showed that the strain expressing the wild-type enzyme had a half-life of 1.5 hours, the DH10b pUC19_MDPE_A23I had a half-life of 6 hours, and the DH10b pUC19_MDPE_G269A had a half-life of 2 hours. In addition, when expressed as a relative percentage based on the half-life 1 (1.5 hours) of the microbial cell producing the wild-type enzyme, the DH10b pUC19_MDPE_A23I had a relative half-life of 4 times and the pUC19_MDPE_G269A had a relative half-life of 1.33 times, confirming that the thermostability of the microbial cell expressing the mutant enzyme was superior to that of the microbial cell expressing the wild-type enzyme.

### Example 5. Second mutation screening and enzyme characterization

### Example 5-1. Second mutation screening

Mutagenesis was performed through amplification (e.g., error prone PCR) of second mutagenesis-causing genes by using the A23I, which was selected as having excellent enzyme activity and thermal stability in Examples 3 and 4, as template DNA. This process results in an additional mutation to the first mutation, A23I, allowing the effect of double mutation to be evaluated.

Specifically, a recombinant vector (pUC19_MDPE_A23I) in which the A23I mutation occurred was prepared by using the pUC19_MDPE substantially the same manner as in Example 2-1 and the MDPE_A23I_cloning primer.

The prepared pUC19_MDPE_A23I was subcloned into pUC19 by inducing an enzyme mutation in substantially the same manner as in the error prone PCR process of Example 2-2, and the subcloned mutant strains were screened by HTS of Example 2-3 for mutant microbial cells with increased activity, and a strain (DH10b pUC19_MDPE_A23I/E267K) in which the A23I and E267K mutations were confirmed was obtained in substantially the same manner as in Example 2-4.

### Example 5-2. Enzyme activity confirmation by using strains producing second-selected mutant enzymes

The wild-type enzyme-producing strain DH10b pUC19_MDPE of Example 1 and the strains DH10b pUC19_MDPE_A23I and DH10b pUC19_MDPE_A23I/E267K, which were confirmed to have enzyme mutations, were tested for their conversion activity in substantially the same manner as in Example 3-2, and the results are shown in Table 6 below.

In Table 6 below, the substrate conversion activity is expressed as relative conversion activity based on 100% of the substrate conversion activity of the strain producing the wild-type enzyme.

**[Table 6]**

| Strain | Relative activity (%) |
|---|---|
| DH10b pUC19_MDPE (wild-type) | 100.00 |
| DH10b pUC19_MDPE_A23I | 162.01 |
| DH10b pUC19_MDPE_A23I/E267K | 167.27 |

As a result of analyzing the conversion activity of the enzyme by using the microbial cell reaction of the enzyme-producing microorganism, it was confirmed that the mutant enzyme with the A23I and E267K double mutations had equivalent or greater enzyme activity compared to the mutant enzyme with only the A23I mutation.

### Example 5-3. Enzyme thermostability confirmation by using strains producing second-selected mutant enzymes

In order to confirm the thermostability of the strains producing the second-selected mutant enzymes, excluding the wild-type enzyme-producing strain with low thermostability, DH10b pUC19_MDPE_A23I and DH10b pUC19_MDPE_A23I/E267K were heat treated at a temperature of 70°C in substantially the same manner as in Example 4, and the degree of enzyme activity was analyzed, and the half-life was calculated to evaluate the thermostability.

As a result of measuring the half-life, the half-life of the DH10b pUC19_MDPE_A23I was measured to be 0.67 hours, and that of the DH10b pUC19_MDPE_A23I/E267 was measured to be 10 hours, confirming that the strain producing the A23I/E267K double mutation mutant enzyme has superior thermal stability.

### Example 6. Confirmation of activity and thermostability of purified enzymes

### Example 6-1. Production of recombinant strain for enzyme purification

For the production of a recombinant strain for enzyme purification, purified plasmid pUC19_MDPE_A23I/E267K was prepared by purifying the DH10b pUC19_MDPE_A23I/E267K recombinant strain of Example 5-1 in substantially the same manner as in Example 2-4.

A gene amplification reaction (e.g., PCR) solution with a final volume of 50 µl containing 10 ng of each of the prepared pUC19_MDPE_A23I/E267K, the pUC19_MDPE of Example 1-2, and the pUC19_MDPE_A23I of Example 2-1, 10 pmol of forward primer (base sequence of SEQ ID NO: 13) for each amino acid residue, 10 pmol of reverse primer (base sequence of SEQ ID NO: 14) for each amino acid residue, 1 µl of phusion polymerase, 10 µl of reaction buffer (5X), and 1 µl of 10 mM dNTP was prepared for each template and subjected to PCR, and the information of primers used is shown in Table 7 below.

**[Table 7]**

| Type | Base sequence (5'->3') | SEQ ID NO: |
|---|---|---|
| Enz_Cloning_ For_Primer | ctttaagaaggagatatacatATGAACATCGG TTGCCACG | 13 |
| Enz_Cloning_ Rev_Primer | GGTGGTGGTGCTCGAGTGCGGCCG CGTGCAGACGGATAGAGTCAAC | 14 |

Specifically, PCR was performed by using the PCR solution on a GeneAmp PCR system 9700 including one cycle at 98 °C (30 seconds), 30 cycles of [98 °C (10 seconds), 55 °C (30 seconds), and 72 °C (2 minutes 30 seconds)], and one cycle at 72 °C (3 minutes), and the gene product obtained by the PCR was subcloned into the pET21a (Novagene) plasmid, which was obtained by treatment with Ndel and Xhol.

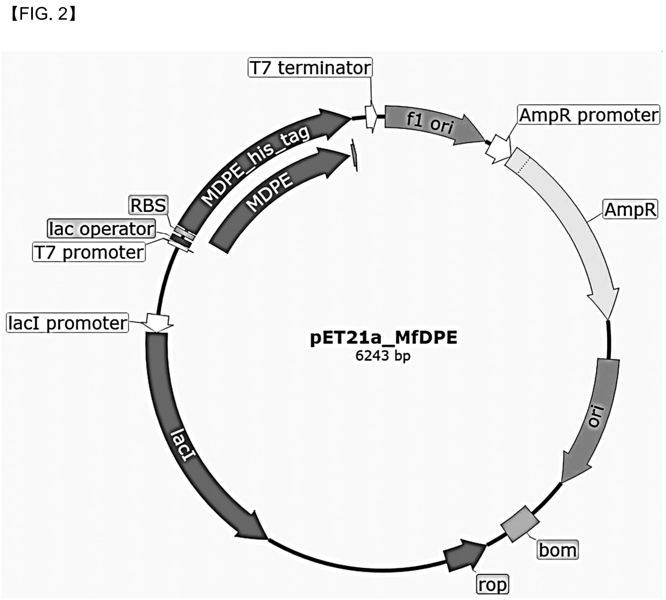

Specifically, to enable expression in the presence of a strong T7 promoter, and to ensure that the amplified gene could be expressed with a His-Tag fusion, the pET21a vector was cut with restriction enzymes Ndel and Xho1 and ligated by using an In-Fusion Snap Assembly kit (Takara) to produce a recombinant vector pET21a/allulose-3-epimerase (pET21a_MDPE). A schematic diagram of the produced recombinant vector is shown in FIG. 2. The produced recombinant vector was transformed into E. *coli* BL21 (DE3) by using a heat shock method to produce a recombinant microorganism (BL21 pET21a_MDPE)).

In addition, PCR reaction was performed in substantially the same manner as in Example 2 by using pET21a_MDPE instead of pUC19_MDPE, and then the PCR-sequenced gene was transformed into E. *coli* BL21 (DE3) by using a heat shock method to produce a recombinant strain.

A strain (BL21 pET21a_MDPE_A23I), in which the A23I mutation was confirmed by using the produced recombinant strain for amino acid residue mutations of the enzyme in substantially the same manner as in Example 2, and a strain (BL21 pET21 a_MDPE_A23I/E267K, in which the A21I and E267K mutations were confirmed in substantially the same manner as in Example 5, were prepared.

### Example 6-2. Enzyme purification

3 mL of each strain, i.e., BL21 pET21a_MDPE, BL21 pET21a_MDPE_A23I, or BL21 pET21a_MDPE_A23I/E267K, was inoculated with colonies into LB-ampicillin liquid medium (supplemented with 10 g/L of Bacto tryptone (Difco), 5 g/L of yeast extract (Difco), 10 g/L of NaCl (Daejung Chemical), and 100 mg/L of ampicillin (Sigma)), and cultured with shaking at 37 °C and 250 rpm until the optical density (OD) at 600 nm reached 1.5. Then, this culture solution was inoculated into 50 mL of the same type of liquid medium as the LB-ampicillin liquid medium and cultured with shaking at 37 °C and 250 rpm.

When the absorbance at 600 nm of the shaken culture solution was 0.5, overexpression of the target enzyme was induced by adding 0.1 mM IPTG. At the point of induction of overexpression, the culture conditions were switched to 20 °C and 180 rpm and maintained for 16 hours. Afterwards, the microbial cells were collected by centrifugation at 4000 rpm for 10 minutes.

50 mL of the recovered microbial cells was dissolved in 1 mL of cooled 50 mM PIPES pH 7.0 buffer solution and then vortexed 5 times for 20 seconds with a bead beater. The cell lysate was then centrifuged at 13,000 rpm at 4 °C for 10 minutes, and only the supernatant was collected.

A Bio-RAD poly-prep chromatography column was filled with 1 ml of Ni-NTA resin, and 10 ml of lysis buffer (50 mM sodium phosphate, 10 mM imidazole pH 8.0), which is 10 times the volume of filled resin, was applied to the column to equilibrate the resin with the buffer, and the collected supernatant was applied to the column. Afterwards, 10 ml of washing I (having the same composition as the lysis buffer solution) containing 50 mM sodium phosphate and 10 mM imidazole pH 8.0 was applied, and 10 ml of washing II containing 50 mM sodium phosphate and 20 mM imidazole pH 8.0, was applied. After application of washing I and washing II, the protein eluate solution was obtained by elution with 3 ml of 50 mM sodium phosphate and 200 mM imidazole pH 8.0, which is three times the volume of the filled resin.

The obtained protein eluate solution was placed in an Amicon centricon and centrifuged at 4,000 rpm for 20 minutes to concentrate the protein. The enzyme was purified by replacing the buffer solution with 50 mM PIPES pH 7.0 in 20 volume folds to the concentrate by repeating a process of filling the concentrate with 20 volume folds of 50 mM PIPES pH 7.0 and centrifuging the filled concentrate at 4,000 rpm four times.

### Example 6-3. Confirmation of purified enzyme activity

The His-tag purified enzyme of BL21 pET21a_MDPE, BL21 pET21a_MDPE A23I, or BL21 pET21a_MDPE_A23I/E267K prepared in Example 6-2 was prepared, and a reaction solution containing 1 mM manganese ions in 50 mM PIPES buffer solution (pH 7.0) so that the concentration of fructose as a reaction substrate was 50 mM was used. When the concentration of the purified enzyme was 0.008 mg/mL each, the reaction was performed at 70 °C for 30 minutes.

Following the enzyme reaction, the mixture was boiled for 5 minutes and centrifuged (13,000 rpm, 15 minutes) to collect the supernatant which was then subjected to HPLC. The HPLC analysis was performed by using a refractive index detector (RID, Agilent 1280 RID) of HPLC (Agilent, USA) equipped with a Cosmosil Sugar D column. Here, the mobile phase solvent used was 80 % (v/v) acetonitrile, the temperature was 30 °C, and the flow rate was 1.0 mL/min. The enzyme activity analyzed by HPLC is shown in Table 8 below.

**[Table 8]**

| Enzyme | Relative activity (%) |
|---|---|
| BL21 pET21a_MDPE (wild-type) | 100 |
| BL21 pET21a_MDPE_A23I | 136.67 |
| BL21 pET21a_MDPE_A23I/E267K | 140 |

As a result of confirming the enzyme activity through a conversion reaction performed with the purified enzyme, the activity increased compared to the wild-type enzyme, similar to the results of the enzyme activity analysis using the microbial cell reaction.

### Example 6-4. Confirmation of thermal stability of purified enzymes

After the purified enzyme of the BL21 pET21a_MDPE or BL21 pET21a_MDPE_A23I/E267K of Example 6-2 was subjected to heat treatment at 70 °C for 30 minutes, 1 hour, 90 minutes, 3 hours, 5 hours, or 7 hours, the activity of residual enzyme was analyzed by using the HPLC analysis method as described in Example 6-3, and the results are shown in Table 9 below. The values listed in Table 9 represent relative activity (%) of the enzyme after heat treatment compared to 100 % of the enzyme activity before heat treatment.

**[Table 9]**

| Relative activity | BL21 pUC19_MDPE (wild-type) | BL21 pUC19_ MDPE_A23I/E267 |
|---|---|---|
| 0 hour heat treatment | 100 | 100 |
| 0.5 hour heat treatment | 70.73 | 95.44 |
| 1 hour heat treatment | 44.78 | 85.47 |
| 1.5 hour heat treatment | 34.92 | 75.87 |
| 3 hour heat treatment | 13.82 | 72.64 |
| 5 hour heat treatment | 10.07 | 66.98 |
| 7 hour heat treatment | 9.81 | 65.57 |

As a result of confirming the thermostability, the half-life of the enzyme activity of the A23I/E267K mutant enzyme could not be determined within the heat treat time (7 hours) due to its excellent thermostability. The wild-type enzyme was confirmed to have a half-life of about 1 hour and 40 minutes, and the A23I/E267K mutant enzyme was confirmed to retain about 60 % or more of its activity even after 7 hours of heat treatment. The half-life of the A23I/E267K mutant enzyme was calculated by using a calibration curve formula and found to be about 9 hours. Through experiments of confirming enzyme activity and thermal stability, it was confirmed that the mutant enzymes were suitable for industrial production of allulose.

## Claims

1. An allulose-3-epimerase protein having a sequence with at least 80 % sequence identity to a sequence in which
at least one amino acid selected from the group consisting of amino acids at positions 23, 75, 115, 126, 255, 267, and 269 from the N-terminus of the amino acid sequence of SEQ ID NO: 1 is substituted with at least one amino acid selected from the group consisting of isoleucine (I), glutamic acid (E), glycine (G), valine (V), alanine (A), and lysine (K),
provided that an enzyme protein having the amino acid sequence of SEQ ID NO: 1 is excluded.

2. The allulose-3-epimerase protein of claim 1, comprising at least
one selected from the group consisting of
a substitution of alanine at position 23 with isoleucine,
a substitution of serine at position 75 with glutamic acid,
a substitution of aspartic acid at position 115 with glycine,
a substitution of asparagine at position 126 with valine,
a substitution of leucine at position 255 with valine,
a substitution of glutamic acid at position 267 with lysine, and
a substitution of glycine at position 269 with alanine,
wherein the positions are numbered from the N-terminus of the amino acid sequence of SEQ ID NO: 1.

3. The allulose-3-epimerase protein of claim 1, wherein the allulose-3-epimerase protein has an allulose conversion activity of 130 % to 200 % relative to the allulose conversion activity of an enzyme comprising the amino acid sequence of SEQ ID NO: 1, which is defined as 100%.

4. The allulose-3-epimerase protein of claim 1, comprising:
at least one amino acid substitution selected from the group consisting of a substitution of alanine at position 23 with isoleucine, a substitution of serine at position 75 with glutamic acid, a substitution of aspartic acid at position 115 with glycine, a substitution of asparagine at position 126 with valine, a substitution of leucine at position 255 with valine, and a substitution of glycine at position 269 with alanine; and
a substitution of glutamic acid at position 267 with lysine,
wherein the positions are numbered from the N-terminus of the amino acid sequence of SEQ ID NO: 1.

5. The allulose-3-epimerase protein of claim 4, wherein the allulose-3-epimerase protein has an allulose conversion activity of 130 % to 200 % relative to the allulose conversion activity of an enzyme comprising the amino acid sequence of SEQ ID NO: 1, which is defined as 100%.

6. The allulose-3-epimerase protein of claim 4, wherein an allulose conversion activity after heat treatment reaction at 70°C for 7 hours is 60% to 100% relative to the allulose conversion activity of the allulose-3-epimerase protein before the heat treatment reaction, which is defined as 100%.

7. A polynucleotide encoding the allulose-3-epimerase protein of any one of claims 1 to 6.

8. A recombinant microorganism comprising a gene encoding the allulose-3-epimerase protein of any one of claims 1 to 6.

9. The recombinant microorganism of claim 8, wherein the microorganism is at least one microorganism selected from the group consisting of a strain of the genus Escherichia, a strain of the genus Bacillus, a strain of the genus Corynebacterium, a strain of the genus Saccharomyces, and a strain of the genus Pichia.

10. A composition for producing allulose, comprising at least one selected from the group consisting of the allulose-3-epimerase protein of any one of claims 1 to 6, a recombinant microorganism expressing the allulose-3-epimerase protein, microbial cells of the microorganism, a lysate of the microbial cells of the microorganism, a culture of the microorganism, and an extract of the foregoing.

11. The composition of claim 10, wherein a half-life of the allulose-3-epimerase protein under heat treatment at 60°C is 130% to 500% relative to the half-life of a wild-type enzyme protein comprising the amino acid sequence of SEQ ID NO: 1, which is defined as 100%.

12. The composition of claim 10, wherein a half-life of the allulose-3-epimerase protein under heat treatment at 60°C is from 1.7 to 50 hours.

13. The composition of claim 10, wherein a half-life of the allulose-3-epimerase protein under heat treatment at 70°C is from 0.5 to 20 hours.

14. The composition of claim 10, wherein the composition further comprises at least one selected from the group consisting of a manganese ion and a cobalt ion.

15. A method of producing allulose, comprising reacting a substrate with at least one selected from the group consisting of the allulose-3-epimerase protein of any one of claims 1 to 6, a recombinant microorganism expressing the allulose-3-epimerase protein, microbial cells of the microorganism, a lysate of the microbial cells of the microorganism, a culture of the microorganism, and an extract of the foregoing.
